**Europäisches Patentamt**

(19) **European Patent Office**

**Office européen des brevets**

(11) Publication number: **0 005 277**

**B1**

(12) **EUROPEAN PATENT SPECIFICATION**

(45) Date of publication of patent specification: **20.01.82**

(51) Int. Cl.³: **C 12 P 7/64**

(21) Application number: **79101406.1**

(22) Date of filing: **08.05.79**

(54) Process for the microbiological production of oil.

(30) Priority: **08.05.78 US 904099**

(43) Date of publication of application:
**14.11.79 Bulletin 79/23**

(45) Publication of the grant of the European patent:
**20.01.82 Bulletin 82/3**

(84) Designated Contracting States:
**BE CH DE FR GB IT NL**

(56) References cited:
**DE - A - 730 231
FR - A - 2 188 963
FR - A - 2 219 223
FR - A - 2 307 472
NL - A - 78 04549**
**CHEMICAL ABSTRACTS, vol. 59, nr. 3, 5th
August 1963, 3104f Columbus, Ohio, USA
M. H. DEINEMA: "Intra- and extra-cellular lipid
production by yeasts".**

**CHEMICAL ABSTRACTS, vol. 66, nr. 25,
19th June 1967, 114598u
Columbus, Ohio, USA**

(73) Proprietor: **CPC INTERNATIONAL INC.
International Plaza
Englewood Cliffs, N.J. 07632 (US)**

(72) Inventor: **Picataggio, Stephen K.
132 Robin Road Bldg. 4, Apt. 52
Somerville New Jersey 08876 (US)**
Inventor: **Smittle, Richard B.
2 Cottage Place
Westfield New Jersey 07090 (US)**

(74) Representative: **Lederer, Franz, Dr. et al,
Patentanwälte Dr. Franz Lederer Dipl.-Ing. Reiner
F. Meyer-Roxlau Lucile-Grahn-Strasse 22
D-8000 München 80 (DE)**

(56) References cited:
**CHEMICAL ABSTRACTS, vol. 76, nr. 9, 28th
February 1972, 43818j Columbus, Ohio, USA
G. H. BELL: "Action of monocarboxylic acids on
Candida tropicalis growing on hydrocarbon
substrates"**

**CHEMICAL ABSTRACTS, vol. 76, nr. 25, 19
June 1972, 152059h Columbus, Ohio, USA**

Courier Press, Leamington Spa, England.

(56) References cited:

PROCESS BIOCHEMISTRY, vol. 9, November
1974, pages 14—22
D. A. WHITWORTH et al.: "Microorganisms as a
Potential Source of Oils and Fats"

0 005 277

## Process for the microbiological production of oil

The supply of naturally-occurring glyceryl-ester oils (by which term it is meant also to include fats) is commonly subject to periods of scarcity which may elevate prices and/or disturb various segments of industry. In addition, the limited variety of such oils restricts their usefulness for many practices. It is therefore understandable that alternative supplies, sources and types of such oils have been investigated extensively.

One of the more promising of these alternatives involves the cultivation of oil-synthesizing microorganisms. Many microorganisms — including algae, bacteria, molds and yeasts — which can synthesize oil in the ordinary course of cellular metabolism have been found. The basic process steps for this means of producing oils are simple and uniform. All that is required is that the microorganism be cultivated in a suitable culture medium to allow synthesis of the oil, then separated from the medium and treated for recovery of its intracellular content of oil.

It is within the framework of these basic steps that most investigation has taken place. In particular, researchers have concentrated on seeking to identify the combinations of specific microorganisms, media, and conditions which would permit economically practicable oil production.

In a paper by Maria H. Deinema "Intra- and Extra-Cellular Lipid Production by Yeasts" published in Mededelingen van de Landbouwhogeschool te Wegeningen, Nederland *61* (2), 1—54 (1961), referred to in Chem. Abstracts, Vol. 59 No. 3, 5th August 1963, p. 3104 f there are described experimental results of fat formation by various microorganisms and it is demonstrated that the addition of fatty acids to the culture medium had a pronounced effect on the composition of the yeast fat.

Despite extensive efforts, however, the most crucial deficiencies of microbiological oil production have remained. With few exceptions, microorganisms have produced disappointing low yields of oil. Even the most successful microorganisms, for example, seldom reached oil contents of as much as 40% by dry cell weight and the great majority had substantially lower yields. This fact, coupled with the difficulties of recovering the intracellularly stored oil, made the cost of this approach generally prohibitive.

The drawbacks imposed by low yields, however, are further compounded by the inability to exert adequate control over the composition of the oil synthesized. If, for example, certain of the known specialty oils or improved oils could be produced, the higher prices they could command might provide commercial justification for this approach. Thus far, however, no such general means of control has been discovered.

In accordance with the present invention, it is possible both to improve the yield of oil synthesized by a microorganism and to exert substantial control over the chemical composition of that oil. By thus permitting high yields of preselected oils of particular importance, this invention renders the microbiological production of oils a commercial practicality.

In its most general embodiment, this invention involves the discovery that the aerobic synthesis of oils by yeasts may be controlled by using certain fatty acids as the carbon nutrient source of their culture or fermentation media. These fatty acids, which have from 10 to 20 carbons, may act individually or collectively to increase oil yield and to cause synthesis of oil having a particular, preselected composition.

In additional embodiments, various preferred media ingredients and cultivation conditions are described. These optional modes of carrying out the process of this invention have been found to complement — and thus still further improve — the yields and control imparted by the present fatty acids.

The microorganisms useful in this process are the oil synthesizing yeasts. Many such yeasts which are naturally capable of oil synthesis are already known and available in the art. Particularly preferred, however, are species from the genus *Rhodosporidium, Lipomyces, Candida, Endomyces, Saccharomyces,* or *Rhodotorula.* Such yeasts are among those which are naturally higher accumulators of oils (or fats).

These oil-synthesizing yeasts are well known and can be isolated by conventional processes from their native sources — such as leaves, vegetable stems or the like, as the case may be. More conveniently, however, they are available from the various culture storage depots (including, for example, the American Type Culture Collection in Maryland, U.S.A.). At such depots it is often possible to obtain even a variety of different cultures of a given yeast species.

While any of the known oil-synthesizing yeasts may be employed in the process of the present invention, it is preferred to utilize those naturally inclined to the synthesis and storage of large amount of oils. Thus, for example, yeasts having the ability to accumulate at least 20% oil (more, desirably at least 30%) on a standard culture medium such as a glucose, ammonium salt, and minerals medium are preferred. They are more susceptible to modification to desired levels of oil synthesis in accordance with the present process.

Illustrative of the available oil-synthesizing yeasts are Rhodosporidium toruloides — ATCC nos. 26194, 26217, 10657, 10788 and 32767

Lipomyces starkeyii — ATCC no. 12659

Lipomyces lipoferus — ATCC nos. 10742 and 32371

Candida revkaufi — ATCC nos. 10570 and 18470

Candida pulcherrima — ATCC nos. 7696, 7697, 9889, 18406 and 22032

Candida tropicalis — ATCC nos. 750, 1369, 9968, 13803, 14056, 14246, 15114, 15543, 18213, 18526, 20005, 20006, 20007, 20115, 20175, 20215, 20240 and 20336

Candida utilis — ATCC nos. 8205, 8206, 9226, 9256, 9950, 15239, 16321, 20262 and 22023 Candida lipolytica — ATCC nos. 8661, 8662, 9773, 16617, 16618, 18942, 18943, 18944, 18945, 20114, 20182, 20237 and 20287

Saccharomyces cereuisae 500 types — ATCC nos. 11795 and 12341 are two examples Rhodotorula glutinis — ATCC nos. 2527, 4054, 15125, 16725, 16726, 16740, 20147 and 20310

Rhodotorula graminis — ATCC nos. 16727, 16728, 16729, 16730 and 18159

Trichosporon pullans — ATCC nos. 9331 and 10677

Trichosporon cutaneum — ATCC nos. 757, 4155, 10267, 13445, 14254, 14255, 14905 and 22375

(It should be noted that the Trichosporon species were formerly known as Endomyces).

The initial selection of a particular yeast species may — to the extent known in the art — suggest various beneficial ingredients for a culture or fermentation medium. If so, such preference may be followed to the extent — described in detail below — that does not conflict with the present invention. In general, however, suitable media may be prepared from any of their common constituents, the only caveat being that a predominent carbon nutrient source must be composed of the fatty acids that control the end product of this invention.

These media, of course, generally are dilute and of aqueous base. Their solid constituents — predominantly carbon and nitrogen nutrient sources — ordinarily compose less than about 6% by total weight. In addition, media are adjusted or buffered to within the pH range of between 4.0 and 6.0. This is the optimum range for yeast cultivation.

As a nitrogen nutrient source for the medium, organic materials such as asparagine, glutamine and peptones are suitable. Alternatively, minerals such as ammonium salts or urea may be employed. Preferably, however, one of the common industrial waste materials rich in nitrogen is employed. Of these, cornsteep is particularly desirable.

Cornsteep is the aqueous liquor formed in the conventional corn-wet-milling process when dry corn is soaked in warm, dilute sulfurous acid. While composed of about 25% by total weight of crude protein (8% nitrogen by dry weight), it also commonly contains small amounts of ash, sugars and other beneficial culture constituents. Although it may be augmented with additional nitrogen-containing materials, cornsteep alone represents an inexpensive, but essentially complete, nitrogen nutrient source.

The culture medium also should include the known essential metabolic mineral salts, including potassium, sodium, calcium, magnesium and iron. Further, such secondary nutrients as vitamins and amino acids are desirable, particularly where the cultivation period is intended to be extensive.

Suitable carbon nutrient sources which may be present in the culture medium are the monosaccharides, disaccharides, oligosaccharides and polysaccharides such as starch hydrolyzates. Alternatively, various diverse sources including hydrocarbons or lower fatty acids including industrial wastes such as cheese production liquors, wood saccharification and waste liquors may be incorporated into the culture medium.

Whether or not such other carbon nutrient sources are provided as a part of the culture medium, however, the culture must contain a predominant amount of one or more fatty acids having from 10 to 20 carbons. This content of fatty acid is necessary because it also serves as the primary means of modifying the oil-synthesis of the yeast cells. Furthermore, because the ability of the fatty acid(s) to act as a modifier of cellular oil synthesis may be partly masked by the presence of other carbon nutrient sources, it is preferred that such other sources be maintained at a level of less than 50% of total carbon source. Most preferably, the fatty acid source constitutes all or essentially all of the available nutrient carbon in the culture medium.

The fatty acid employed as the carbon source may be obtained from any number of known sources and/or be of a variety of compositions. Most simply, for example palmitic ($C_{16:0}$), stearic ($C_{18:0}$) or oleic ($C_{18:1}$) fatty acid is generally available commercially in either free acid or simple salt — such as sodium salt — form of high purity. These more prevalent fatty acids are therefore readily employed either alone or as admixtures. Polyunsaturated fatty acids — especially linoleic ($C_{18:2}$) and linolenic ($C_{18:3}$) — and other less common fatty acids having 16 to 20 carbons may also be obtained in pure form, but are available in greater and more inexpensive quantities in various known commercial admixtures such as soapstock, discussed below.

Although the composition of the carbon source is of only minor concern as regards its nutrient function, that composition becomes of major importance in fulfilling the second function of the fatty

4

acids — *viz.*, modification and control over oil-synthesis occurring within the yeast cells. Here the specific fatty acid or admixture is particularly vital to preselection of the chemical composition of the product oil and also to the degree of increase from conventional oil yields.

The importance of this fatty acid composition in the culture medium stems from the fact that each individual fatty acid causes a different kind of shift in the oil-synthesizing metabolism of a given yeast species. Moreover, the result of admixtures of fatty acids is a collective interaction which compounds — but not necessarily in linear fashion — these individual shifts. Hence, given product oils are chiefly produced through orchestration of a proper mix of fatty acids in the carbon source.

In view of the complexity of their mode of modification of oil-synthesis, accurate prediction of the precise yield and oil composition to be obtained from any given fatty acid carbon source must be at least partly empirically-based. This does not, however, present any serious impediment to the practice of this invention. Conventional analytical practices permit determination of the yield and composition of oil resultant from any particular culture. Hence, routine experimentation will soon permit identification of fatty acid carbon sources suitable for the production of any given oil.

Furthermore, even the degree of experimentation involved may be reduced by the fact that certain general rules as to the effect of the fatty acids on oil-synthesis exist. For example, the presence of fatty acid of any given carbon length in the carbon source ordinarily results in an increase in the proportion of esters of that same length in the oil. Similarly, the degree of saturation/unsaturation (and particularly polyunsaturation) in product oil is directly related to the corresponding saturation level in the fatty acid composition of the carbon source.

As will become more evident from the further description and examples which follow, such general rules — while helpful — are incomplete. Variation in desired oil, yeast species, media constituents and cultivation conditions all exert some influence over oil production. Because, however, the major modifying effect derives from these carbon source fatty acids of from 10 to 20 carbon, it is relatively easy to approximate any desired kind of oil production. Further accuracy may then be obtained through such secondary modifying factors so as to provide a complete set of the parameters necessary for preselected and optimal operation of a given, desired oil.

Among the secondary factors discovered to affect oil-synthesis, two are of particular importance. These factors relate to the state of the yeast cells themselves and to the conditions under which they are cultivated.

The metabolic state of a yeast cell, for example, may be considered to be balanced between two major avenues. On the one hand, the cell may be in a growth stage in which production of protein etc. is the predominant activity. Alternatively, the cell may be relatively inert as to growth and instead concentrate upon synthesis and storage of fat. Obviously, however, the latter state of metabolic activity is more desirable where high yield of oil is sought.

Fortunately, it has been discovered that the cellular state of a yeast depends upon the composition of the medium in which it is being cultivated. More specifically, for example, it is possible to favor the production of oil through the expedient of minimizing the amount of nitrogen nutrient source in the medium. Some nitrogen source — the amount depending largely on the yeast species being cultivated — is always necessary. However, by reducing its content to, for example, the range of between about .005 to 0.2% by nitrogen weight (as compared to the preferred range of 0.5 to 5.0% by carbon weight for the carbon source) further improvement in yield may be obtained.

In a similar manner, the content of dissolved oxygen in the medium may affect the yeast cells directly. Whether or not such effect results from cellular oxidation of stored oil, aerobic cultivation of these yeasts at different levels of oxygen in the culture medium may substantially increase the final yield of recovered oil.

In contrast to the above, the effects of the conditions of yeast cultivation are also reflected in the composition of the resultant oils. Here, temperature is significant. While desirable cultivation may ordinarily occur throughout the range of from 20 to 40°C, it has been discovered that from 30 to 40°C favors the production of saturated oils, while at 20 to 30°C the oil synthesized is relatively more unsaturated in its fatty acid constituents. In addition to this compositional effect, however, some increase in yield with elevated temperatures is also common.

Through use of the foregoing means for modifying oil-synthesis in yeast, various types of particularly useful and/or scarce oils may be produced. For example, an especially desirable product is an oil containing a substantial proportion of disaturated, unsaturated triglyceride. The value of, for example, cocoa butter lies largely in its content of such an oil (in which the unsaturated acid generally occupies the middle position).

This invention thus offers a means of producing high yields of oils tailored to meet particular demands. For example a functional cocoa butter substitute can be produced through microbiological means. As has already been indicated, the fatty acid and optional modifying means of this invention readily permit control over the degree of satuation in product oils. The ratio of saturated-to-unsaturated acid constituents in such glyceryl oils may be readily controlled to achieve the approximate level of 2.0 thus approximating cocoa butter. Similarly, assurance of high contents of palmitic, oleic and stearic radicals (as normally are predominant in cocoa butter) is easily afforded by selection of a carbon source rich in these acids.

5

Other valuable oils readily produced in accordance with the present invention are oils high in polyunsaturates. By selection of a carbon source which is itself rich in polyunsaturated acids, such an oil is readily produced. Use of such polyunsaturates readily permits production of oils having a polyunsaturated/saturated ratio of 2.0 or more. Moreover, for this purpose, one of the previously mentioned commercial admixtures such as soapstock (a waste product of vegetable oil refining which is particularly rich in linoleic and linolenic acids) may be utilized as all or a part of the fatty acid carbon source. This then substantially reduces the cost of ingredients utilized in the oil synthesis.

Once the yeast cells have been cultivated for the desired period of time (ordinarily between from about 1 to 7, more desirably 2 to 5, days), the medium should be separated. This may be accomplished by any of the conventional means such as filtration or centrifugation. Additionally, improved separation may be afforded by washing with water or the like, as desired.

After separation of the cells, their oil content is removed. Again, conventional means may be employed. Preferably, however, recovery follows a two-step pattern in which the cells are first subjected to rupture by, for example, freezing or hydrolysis and then the oil is extracted from the cellular debris with a suitable (usually volatile, to facilitate subsequent removal) solvent. In a further embodiment, the cell debris may also be dried prior to extraction or during rupture, as with freeze-drying. This further improves the purity of the recovered oil.

Further illustrative of the present invention are the examples which follow. In the examples, all proportions are on a weight basis unless otherwise indicated.

Example 1

Ten culture flasks were filled with growth media containing 1.0% cornsteep and 2.0% sodium salt compositions of various fatty acids. The acids of these salts were as follows:

| Media/Flask | Fatty Acid Composition |
|---|---|
| 1 | 2.0% palmitic (i.e., sodium palmitate) |
| 2 | 2.0% stearic (i.e., sodium stearate) |
| 3 | 2.0% oleic (i.e., sodium oleate) |
| 4 | .66% palmitic; .66% stearic; .66% oleic |
| 5 | 1.0% palmitic; 1.0% stearic |
| 6 | 1.0% stearic; 1.0% oleic |
| 7 | 1.0% palmitic; 1.0% oleic |
| 8 | 1.33% palmitic; .33% stearic; .33% oleic |
| 9 | .33% palmitic; 1.33% stearic; .33% oleic |
| 10 | .33% palmitic; .33% stearic; 1.33% oleic |

Each of the media was adjusted to a pH of 5.0, inoculated with *Rhodosporidium torulodies* (ATCC 10788), placed in an aerated shake incubator, and there maintained at 27°C under agitation for 7 days. The yeast cells were then harvested by centrifugation washed with cold, sterile water dried by recentrifugation and the cells were frozen until their oil content could be analyzed.

| Sample No. | 1 | 2 | 3 | 4 | 5 | 6 | 7 | 8 | 9 | 10 |
|---|---|---|---|---|---|---|---|---|---|---|
| Lipid Content (%) | 66 | 60 | 47 | 66 | 67 | 65 | 72 | 79 | 70 | 67 |
| Fatty Acids | | | | | | | | | | |
| C 14:0 | 1.3 | 1.7 | 1.5 | 1.5 | 1.1 | 1.6 | 1.9 | 1.9 | 1.8 | 1.7 |
| C 16:0 | 62.5 | 39.7 | 6.8 | 52.8 | 48.1 | 39.2 | 54.2 | 71.4 | 42.1 | 17.7 |
| C 16:1 | | 0.7 | | | 0.3 | 1.1 | | | | |
| C 18.0 | 12.2 | 38.9 | 1.0 | 19.4 | 27.3 | 33.7 | 1.3 | 9.7 | 27.2 | 2.4 |
| C 18:1 | 22.6 | 16.9 | 67.3 | 18.1 | 16.6 | 22.3 | 40.2 | 15.4 | 21.0 | 64.5 |
| C 18:2 | 1.0 | 1.6 | 5.2 | 0.7 | 0.9 | 1.0 | 1.9 | 1.0 | 1.5 | 3.0 |
| C 18:3 | | | 1.0 | 0.2 | | 0.3 | | | 0.6 | 0.6 |
| Saturated | 76 | 80 | 9 | 74 | 77 | 75 | 57 | 83 | 71 | 22 |
| Monounsaturated | 23 | 18 | 67 | 18 | 17 | 23 | 40 | 15 | 21 | 65 |
| Polyunsaturated | 1 | 2 | 6 | 1 | 1 | 1 | 2 | 1 | 2 | 4 |
| Unknown | 0 | 0 | 18 | 7 | 5 | 1 | 1 | 1 | 6 | 9 |
| Iodine Value | 21.2 | 17.8 | 69.6 | 17.3 | 16.1 | 22.6 | 37.8 | 15.0 | 22.7 | 62.2 |

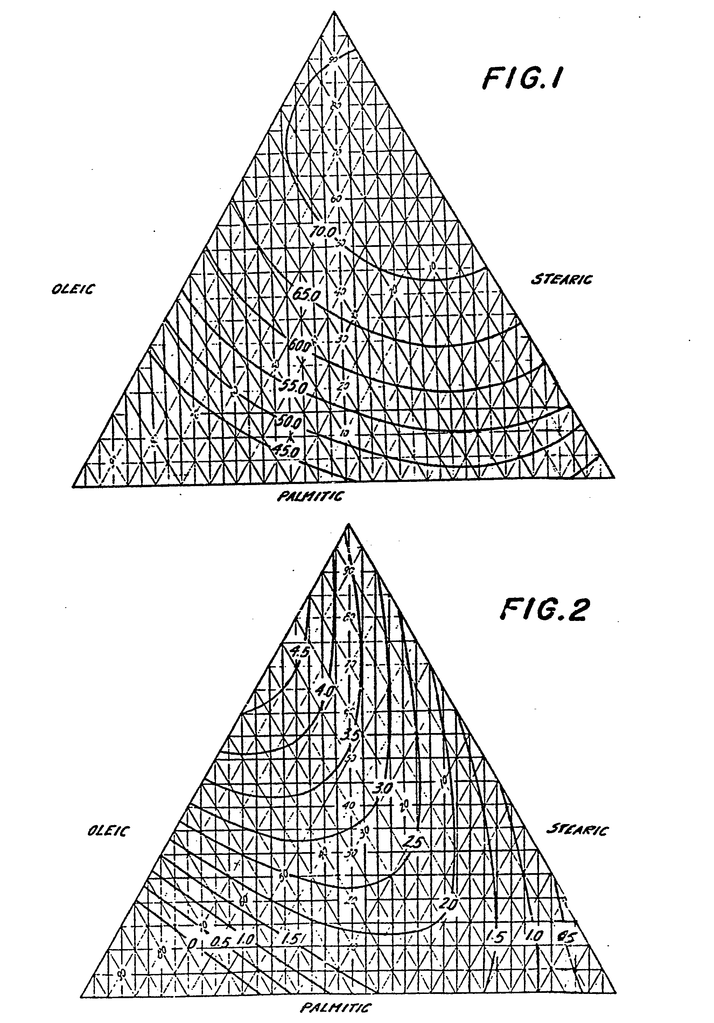

The yields of oil (provided on the basis of percent by total dry cell weight) from the different media are depicted graphically in FIG 1 of the drawings. The three axes of the figure represent the relative amounts of palmitic, stearic and oleic acids in the 2.0% fatty acid fraction of the growth medium. There the effect of the differing fatty acid carbon sources or modifying agents are seen to be considerable. The weight percent of oil in the cultivated cells varies from less than 45% to more than 70% by weight, dependent solely upon the composition of the fatty acids present in the media.

In FIG 2, the effect of the different carbon sources upon the composition of the oil itself is depicted. This graph charts the changes in the composition of the yeast lipid as measured by the ratio of saturated-to-unsaturated fatty acid constituents thereof as a function of the substrate composition on which the yeast is grown. Again the effect is considerable. Moreover, the graphed results depend solely upon the degree of saturation/unsaturation of the fatty acids of the media carbon sources showing that the individual fatty acid directly influence the nature of the metabolic functions of the yeast cells.

Example 2

A set of 3 liter samples of 4 different media were inoculated to a 2% level with *Rhodosporidium torulodies* (ATCC 10788). The set contained a control media of 1.0% cornsteep and 2.0% soapstock. The remaining 3 samples in the set contained 1.0% cornsteep, 1.5% soapstock and 0.5% of one of palmitic acid, stearic acid and oleic acid, respectively.

All samples were incubated for 3 days under aeration and agitation at 25°C. The yeast cells were then treated as in Example 1 for recovery of their oil content. The analyses of the oil contents of these samples are depicted below in conjunction with the corresponding fatty acid carbon sources of the various media.

This data underlines the complexity of action of the fatty acid carbon sources. Illustrative is the divergence in effect between the oleic (#4) and palmitic (#2) enriched sources versus the stearic (#3) enriched one. Modification of oil composition of the former class proceeds with large increases in the presence of the enhancing fatty acid as compared to the control of soapstock (#1). With stearic acid, however, the oil synthesized shows a much smaller increase in that acid which may be due to metabolization of that acid or a feedback inhibition mechanism.

| Fatty Acids | Media Composition | | | | Oil Composition (3 day culture) | | | |
|---|---|---|---|---|---|---|---|---|
| | #1 | #2 (16:0) | #3 (18:0) | #4 (18:1) | #1 | #2 (16:0) | #3 (18:0) | #4 (18:1) |
| C 14:0 | 0.1 | 0.1 | 0.1 | 0.1 | 0.3 | 0.9 | 1.2 | 0.6 |
| 16:0 | 17.4 | 37.9 | 12.9 | 12.9 | 14.0 | 38.7 | 31.8 | 13.4 |
| 16:1 | 0.2 | 0.2 | 0.2 | 0.2 | 0.7 | — | 2.2 | 8.9 |
| 18:0 | 4.6 | 3.3 | 28.3 | 3.3 | 0.8 | 0.2 | 9.8 | — |
| 18:1 | 24.0 | 18.0 | 18.0 | 43.0 | 31.8 | 24.6 | 39.0 | 53.2 |
| 18:2 | 22.5 | 16.8 | 16.8 | 16.8 | 44.4 | 30.0 | 12.1 | 21.8 |
| 18:3 | 26.2 | 19.6 | 19.6 | 19.6 | 7.0 | 4.9 | — | 2.1 |
| Saturated | 22.1 | 41.3 | 41.3 | 16.3 | 15.1 | 39.8 | 43.6 | 14.0 |
| Monounsaturated | 24.2 | 18.2 | 18.2 | 43.2 | 32.5 | 24.6 | 41.2 | 62.1 |
| Polyunsaturated | 48.7 | 36.4 | 36.4 | 36.4 | 51.4 | 34.9 | 12.1 | 23.9 |

### Example 3

Four culture flasks containing growth media having a soapstock/cornsteep base were prepared. These media differed only in the ratio of these two constituents. The flasks were inoculated with *Lipomyces starkeyi* to a 2% level and cultivated for 7 days as in Example 1.

Analysis of the oil recovered from the yeast showed the following:

| | Sample (Cornsteep/soapstock) | | | |
| | (0.5%/2.5%) | (1.3%/1.7%) | (2.1%/0.9%) | (2.5%/0.5%) |
|---|---|---|---|---|
| Lipid Content | 31.3% | 51.8% | 67.3% | 39.1% |
| Cell growth per ml* | $3.4 \times 10^7$ | $2.6 \times 10^7$ | $2.1 \times 10^7$ | $1.6 \times 10^7$ |
| Fatty Acids | | | | |
| C 14:0 | 0.2 | 0.1 | 0.1 | 0.2 |
| C 16:0 | 21.1 | 19.6 | 19.8 | 21.1 |
| C 16:1 | 0.4 | 0.2 | 0.4 | 0.6 |
| C 18:0 | 4.7 | 4.3 | 2.6 | 1.9 |
| C 18:1 | 12.4 | 13.8 | 24.2 | 29.7 |
| C 18:2 | 52.2 | 53.8 | 36.4 | 31.5 |
| C 18:3 | 7.2 | 6.4 | 14.8 | 13.9 |
| Polyunsaturated | 59.4% | 60.2% | 51.2% | 45.4% |
| Monounsaturated | 12.8% | 14.0% | 24.6% | 30.3% |
| Saturated | 26.1% | 24.6% | 22.8% | 23.5% |

*as determined by Petroff-Hauser method

The table shows the strong influence of variation in the amount of fatty acid carbon source or modifying agent present in the culture medium. This is particularly true with regard to the fatty acid profile of the glyceryl esters themselves.

The differing oil-yield and cellular-growth values reflect the composite result of two factors. While higher concentrations of carbon source tend to stimulate production of oil *per se*, significant amounts of the nitrogen-rich cornsteep can cause a shift in cellular metabolism toward cell growth and away from oil production. This shift is reflected in the subsequent decline in yield.

### Example 4

*Candida utilis* was cultivated in two media differing by the presence of cornsteep and soapstock in amount of 0.5%/2.5% and 2.5%/0.5% by weight, respectively. Culture and recovery were as set forth in Example 3 except that a 7 day growth period was employed.

The results were similar to those obtained in Example 3. At the higher level of fatty acid carbon source, the yield of oil was 58.2% with a corresponding growth factor of $1.2 \times 10^8$/ml. With the higher ratio of nitrogen-containing cornsteep, however, the growth factor increased to $1.5 \times 10^8$/ml and oil production declined to 37.7%. Thus the desirability of controlling growth so as to favor oil production is again emphasized.

9

**0 005 277**

### Example 5

Two growth media were prepared which differed only in their contents of cornsteep and soapstock. The first contained 0.5% cornsteep and 2.5% soapstock; the second, 0.9% and 2.1%, respectively. Yeast of the species *Saccharomyces cerevisiae* was grown and harvested after 7 days as in Example 4.

Analysis of the recovered oils showed the following differences in major constituents.

| | Culture Medium (cornsteep/soapstock) | |
| --- | --- | --- |
| | 0.5%/2.5% | 0.9%/2.1% |
| Fatty Acids | | |
| C 16:0 | 13.9% | 22.1% |
| C 18:0 | 1.8% | 3.0% |
| C 18:1 | 13.7 | 17.4% |
| C 18:2 | 59.2% | 49.7% |
| Polyunsaturated | 66.7% | 53.9% |
| Monounsaturated | 14.8% | 18.4% |
| Saturated | 15.9% | 25.7% |

### Example 6

*Rhodosporidium torulodies* (ATCC 10788) was cultured in two media containing 2.0% soapstock for 7 days. The first sample, however, was cultivated at 25°C and the second at 35°C.

After recovery of the synthesized oil, analysis showed the following significant differences:

| | Cultivation Temperature | |
| --- | --- | --- |
| | 27°C | 37°C |
| Lipid Content | 53.3% | 64.2% |
| Fatty Acid Composition | | |
| C 16:0 | 27.4% | 29.4% |
| C 18:0 | 5.1% | 5.1% |
| C 18:1 | 11.2% | 9.7% |
| C 18:2 | 50.0% | 48.6% |
| C 18:3 | 3.5% | 3.6% |
| Saturated | 34.0 | 35.4 |
| Monounsaturated | 12.0 | 11.0 |
| Polyunsaturated | 53.5 | 52.2 |
| Unknown | 0.5 | 1.4 |

This illustrates the effect of temperature upon the yield in synthesized oil.

10

**0 005 277**

Example 7

Two 3 l samples of a 2.0% soapstock and 1.0% cornsteep medium were adjusted to a pH of 4.5 and inoculated with *R. torulodies* (ATCC 10788). Cultivation was then performed at 25°C for 72 hours, with the distinction that in one sample the oxygen level was maintained at 30% of saturation while in the other, it was maintained at 80%.

After the recovery of the synthesized oil, analysis showed the following significant differences:

|  | 80% $O_2$ | 30% $O_2$ |
|---|---|---|
| Lipid Content | 39.4% | 47.4% |
| Oil Composition | | |
| C 16:0 | 10.1% | 12.4% |
| C 18:0 | 1.7% | 1.9% |
| C 18:1 | 15.5% | 14.5% |
| C 18:2 | 66.7% | 63.7% |
| C 18:3 | 5.5% | 5.3% |

The available oxygen content is thus seen to have a major effect on the yield of oil and a minor one on the degree of saturation, particularly polyunsaturation, of the product oil.

**Claims**

1. A process for the microbiological production of oils comprising
   (a) preparing a growth medium in which the carbon nutrient source comprises fatty acids having from 10 to 20 carbons,
   (b) inoculating said growth medium with oil synthesizing yeast cells,
   (c) aerobically cultivating said yeast cells,
   (d) separating the cultivated cells from said growth medium, and
   (e) recovering oil from the cultivated cells,
characterized in that said growth medium is maintained at a pH between 4.0 and 6.0 and a temperature between 20°C and 40°C and the composition of the oil synthesized is dependent upon the ratio of saturated fatty acid to unsaturated fatty acid in the medium.

2. The process of Claim 1, characterized in that the fatty acid carbon source is between 0.5 and 5.0% carbon by weight of medium.

3. The process of Claim 2, characterized in that the medium contains a nitrogen source of between .005 and 0.2% nitrogen by weight of medium.

4. The process of Claim 3, characterized in that the oil-synthesizing yeast is a species selected from the genus *Rhodosporidium, Lipomyces, Candida, Saccharomyces, Endomyces* or *Rhodotorula*.

5. The process of Claim 3, characterized in that the nitrogen source comprises corn steep.

6. The process of Claim 1, 4 or 5, characterized in that the growth medium is enriched with a fatty acid selected from the group consisting of palmitic acid, stearic acid, oleic acid and combinations thereof.

7. The process of Claim 1, 4 or 5, characterized in that the fatty acid component in the growth medium is comprised predominantly of soapstock.

8. The process of Claim 6, characterized in that the cultivated cells yield at least 50% oil by dry cell weight.

**Revendications**

1. Procédé pour la production microbiologique d'huiles, comprenant les opérations consistant à:
   (a) préparer un milieu de croissance dans lequel la source d'aliments carbonés comprend des acides gras contenant 10 à 20 atomes de carbone,
   (b) ensemencer ce milieu de croissance avec des cellules d'une levure synthétisant l'huile,
   (c) cultiver dans des conditions aérobies ces cellules de levure,
   (d) séparer les cellules cultivées du milieu de croissance et
   (e) récupérer l'huile à partir des cellules cultivées,
caractérisé en ce que le milieu de croissance est maintenu à un pH compris entre 4,0 et 6,0 et à une température comprise entre 20°C et 40°C, et en ce que la composition d l'huile synthétisée dépend du rapport des acides gras saturés aux acides gras insaturés dans le milieu.

11

2. Procédé selon la revendication 1, caractérisé en ce que la proportion de source de carbone sous forme d'acides gras est comprise entre 0,5 et 5,0% de carbone par rapport au poids du milieu.

3. Procédé selon la revendication 2, caractérisé en ce que le milieu contient une source d'azote dans une proportion comprise entre 0,005 et 0,2% d'azote par rapport au poids du milieu.

4. Procédé selon la revendication 3, caractérisé en ce que la levure synthétisant l'huile est une espèce choisie dans le genre *Rhodosporidium, Lipomyces, Candida, Saccharomyces, Endomyces* ou *Rhodotorula.*

5. Procédé selon la revendication 3, caractérisé en ce que la source d'azote comprend de la liqueur de macération du maïs.

6. Procédé selon la revendication 1, 4 ou 5, caractérisé en ce que le milieu de croissance est enrichi avec un acide gras choisi dans le groupe constitué par l'acide palmitique, l'acide stéarique, l'acide palmitique et des combinaisons de ces acides.

7. Procédé selon la revendication 1, 4 ou 5, caractérisé en ce que l'ingrédient introduisant les acides gras dans le milieu de croissance est constitué principalement par de la matière première pour savon.

8. Procédé selon la revendication 6, caractérisé en ce que les cellules cultivées donnent au moins 50% d'huile par rapport au poids des cellules sèches.

**Patentansprüche**

1. Ein Verfahren für die mikrobiologische Herstellung von Oelen durch
a) Herstellung eines Nährmediums, worin die Kohlenstoffquelle Fettsäuren mit 10 bis 20 Kohlenstoffatomen enthält,
b) Beimpfen dieses Nährmediums mit ölsynthetisierenden Hefezellen,
c) Kultivieren dieser Hefezellen unter aeroben Bedingungen,
d) Abtrennen der kultivierten Zellen vom Nährmedium und
e) Gewinnung von Oel aus den kultivierten Zellen,
dadurch gekennzeichnet, dass das Nährmedium bei einem pH-Wert zwischen 4,0 und 6,0 und einer Temperatur zwischen 20 und 40°C gehalten wird und dass die Zusammensetzung des synthetisierten Oels vom Verhältnis von gesättigen Fettsäuren zu ungesättigten Fettsäuren im Medium abhängig ist.

2. Verfahren nach Anspruch 1, dadurch gekennzeichnet, dass die Fettsäurekohlenstoffquelle zwischen 0,5 und 5,0% Kohlenstoff, bezogen auf das Medium, enthält.

3. Verfahren nach Anspruch 2, dadurch gekennzeichnet, dass das Medium eine Stickstoffquelle enthält, die zwischen 0,005 und 0,2% Stickstoff, bezogen auf das Gewicht des Mediums, enthält.

4. Verfahren nach Anspruch 3, dadurch gekennzeichnet, dass die ölsynthetisierende Hefe eine Spezies aus dem Genus *Rhodosporidium, Lipomyces, Candida, Saccharomyces, Endomyces* oder *Rhodotorula* ist.

5. Verfahren nach Anspruch 3, dadurch gekennzeichnet, dass die Stickstoffquelle Kornlauge enthält.

6. Verfahren nach Anspruch 1, 4 oder 5, dadurch gekennzeichnet, dass das Nährmedium mit Fettsäure aus der Gruppe von Palmitinsäure, Stearinsäure, Oelsäure oder deren Kombinationen angereichert ist.

7. Verfahren nach Anspruch 1, 4 oder 5, dadurch gekennzeichnet, dass die Fettsäurekomponente im Nährmedium hauptsächlich Soapstock enthält.

8. Verfahren nach Anspruch 6, dadurch gekennzeichnet, dass die kultivierten Zellen mindestens 50% Oel, bezogen auf das trockne Zellgewicht, ergeben.

0 005 277

FIG.1

OLEIC          STEARIC

PALMITIC

FIG.2

OLEIC          STEARIC

PALMITIC

1